# EUROPEAN PATENT APPLICATION

(11) **EP 4 052 753 A1**
(43) Date of publication of application: **07.09.2022**
(21) Application number: 20882297.3
(22) Date of filing: 30.10.2020
(51) Int. Cl.: A61M 37/00, A61K 9/00, A61K 38/48, A61K 47/26, A61K 47/36, A61K 47/32

(54) **MICROSTRUCTURE FORMULATION TECHNIQUE FOR BOTULINUM TOXIN**

(30) Priority: 31.10.2019 KR 20190137301
(71) Applicant: Hugel Inc., Gangwon-do 24206 (KR); Small'Lab Co., Ltd., Daejeon 34-027 (KR)
(72) Inventor: MOON, Kyeong Yeop, Chuncheon-si Gangwon-do 24206 (KR); LEE, Chang Jin, Seoul 04187 (KR); KIM, Dae Gun, Yongin-si Gyeonggi-do 17072 (KR); OH, Dong Hoon, Chuncheon-si, Gangwon-do 24419 (KR); KANG, Do Hyun, Chuncheon-si Gangwon-do 24222 (KR); LEE, Woo Ran, Chuncheon-si Gangwon-do 24326 (KR); SHIN, Juhyung, Suwon-si Gyeonggi-do 16457 (KR); LEE, Jeong Gyu, Daejeon 34049 (KR); YOON, Myeonghee, Daejeon 34027 (KR)
(74) Representative: Schnappauf, Georg
(86) International application number: PCT/KR2020/015063
(87) International publication number: WO 2021/086123

(57) **Abstract**

The present invention relates to a microstructure formulation technique for botulinum toxin. The microstructure formulation technique for botulinum toxin according to the present invention enables the concentration of botulinum toxin to be finely adjusted, alleviates pain when administered into the human body, and enables the toxin to be injected at an accurate administration position. Thus, the technique is expected to be greatly utilized in the safe and convenient medical use of botulinum toxin.

## Description

### [Technical Field]

The present invention relates to a microstructure formulation technique for botulinum toxin.

### [Technical Field]

Various *Clostridium* strains that secrete toxins having a neurotoxic effect have been discovered from the 1890s up to now, and the toxins secreted by these strains has been characterized over the past 70 years (Schant, E. J. et al., Microbiol. Rev., 56:80, 1992). Among these, botulinum toxin is classified into 7 types, including types A to G, according to serological characteristics, and causes systemic asthenia by inhibiting acetylcholine exocytosis at the cholinergic presynapse of the neuromuscular junction in animals having nerve function. It has been known that botulinum toxin types B, D, F and G cleave synaptobrevin, types A and E cleave SNAP25, and type C cleaves syntaxin at specific sites. Therefore, recently, efforts have been made to use the neurotoxicity of botulinum toxin for cosmetic or therapeutic purposes. Technology for using botulinum toxin was suggested or attempted for treatment of various autonomic nerve system disorders including ophthalmic diseases, pain and hyperhidrosis, migraine pain, post-operative pain and visceral pain, psoriasis and dermatitis, various types of cancer, and neurogenic inflammation.

However, botulinum toxin is the most lethal material among known biotoxins, and a half lethal dose for human is 1.3 to 2.1 ng/kg in the case of intravenous injection or intramuscular injection, and 10 to 13 ng/kg in the case of inhalation. As such, since botulinum toxin has a large therapeutic effect on various diseases, but has high toxicity and thus is fatal in a very small amount, when the toxin is used in a living body, technology for fine concentration control and technology for administration to an exact location are essentially required.

Meanwhile, microstructures include a microneedle, a microblade, a microknife, a microfiber, a microspike, a microprobe, a microbarb, a microarray or a microelectrode, and among these, the microneedle refers to a technique to increase drug penetration by puncturing the skin with fine needles. Particularly, when botulinum toxin is delivered using microneedles, the toxin is injected with tens or hundreds of fine needles, so the pain caused by injection may be reduced, and the side effects caused by the failure to administer at an exact location may overcome.

Therefore, the present invention relates to a microstructure formulation technique for botulinum toxin, which uses a microstructure of the present invention to reduce pain during the administration of botulinum toxin and facilitate the administration of a small amount of toxin at an exact location. Therefore, the present invention is expected to be broadly applied for safe and convenient medical use.

### [Disclosure]

### [Technical Problem]

The present invention is provided to solve the above-described conventional technical problems, and provides a microstructure formulation technique for botulinum toxin.

However, technical problems to be solved in the present invention are not limited to the above-described problems, and other problems which are not described herein will be fully understood by those of ordinary skill in the art from the following descriptions.

### [Technical Solution]

Hereinafter, the present invention will be described with reference to various embodiments. In the following description, for full understanding of the present invention, various specific details, for example, specific forms, compositions and processes will be described. However, certain embodiments may be implemented without one or more of the specific details, or in combination with other known methods and forms. In another example, known processes and manufacturing techniques are not described in particular detail so as to not unnecessarily obscure the present invention. Throughout the specification, the reference to "one embodiment" or "embodiment" means that a specific feature, form, composition or property described in conjunction with the embodiment is included in one or more embodiments of the present invention. Therefore, the context of the "one embodiment" or "embodiment" expressed in various locations throughout the specification does not necessarily represent the same embodiment of the present invention. In addition, a specific feature, form, composition or property may be combined in any suitable manner in one or more embodiments.

Unless specially defined otherwise, all technical and scientific terms used in the specification have the same meanings as commonly understood by one of ordinary skill in the art to which the present invention belongs.

In one embodiment of the present invention, the "botulinum toxin" is a neurotoxin protein produced by *Clostridium botulinum.* There are 127 species or more in the genus Clostridium, which are classified according to a shape and a function. *Clostridium botulinum,* which is an anaerobic, gram-positive bacterium, produces botulinum toxin, a strong polypeptide neurotoxin, causing a neuroparalytic disease called botulism in humans and animals. *Clostridium botulinum* spores are found in soil, and can be cultured in a home-canned food container, which is poorly sterilized and sealed, resulting in a lot of botulism. Symptoms of botulism typically appear 18 to 36 hours after eating food infected with *Clostridium botulinum* culture or spores. Botulinum toxin seems to pass through the intestinal lining without reducing toxicity and exhibits high affinity for cholinergic motor neurons. Symptoms of botulinum toxin poisoning may lead to gait disturbance, dysphagia and speech impairment, respiratory muscle paralysis, and death.

Botulinum toxin type A is the most fatal natural biologic known to man. The LD50 of commercially available botulinum toxin type A (purified neurotoxin complex) is approximately 50 picograms (that is, 1 unit). Interestingly, on a molar basis, botulinum toxin type A is 1.8 billion times more lethal than diphtheria, 600 million times more lethal than sodium cyanide, 30 million times more lethal than cobra toxin, and 12 million times more lethal than cholera. One unit (U) of botulinum toxin may be defined as the LD50 via intraperitoneal injection into female Swiss Webster mice each having a weight of 18 to 20 g.

Generally, 7 immunologically distinct botulinum neurotoxins are characterized by neurotoxin serotypes A, B, C1, D, E, F and G, respectively, which are distinguished by neuralization with type-specific antibodies. The different serotypes of botulinum toxins differ depending on the animal species on which the toxins act and the degree and duration of paralysis caused by the toxins. For example, when measured by the rate of paralysis occurring in rats, botulinum toxin type A was measured to be 500 times stronger than botulinum toxin type B. In addition, botulinum toxin type B was measured to be non-toxic even when administered to primates at 480 U/kg, approximately 12 times the primate LD50 of botulinum toxin type A. Botulinum toxin is considered to bind to cholinergic motor neurons with strong affinity, enter the neurons and inhibit the release of acetylcholine. It can be further taken up not only by phagocytosis and pinocytosis, but also through a low affinity receptor.

Regardless of the serotype, the molecular mechanisms of toxin poisoning are similar, and each seems to include at least three steps. In the first step of the process, by the specific interaction between the heavy chain (H chain or HC) of a toxin and a cell surface receptor, the toxin binds to the presynaptic membrane of the target neuron, and this receptor is considered to be different for each botulinum toxin type and tetanus toxin type. The carboxyl-end fragment and Hc of the heavy chain are considered to be important for targeting of toxins to a cell surface.

In the second step, the toxin crosses the plasma membrane of the poisoned cell. Primarily, botulinum toxin is enclosed by cells through receptor-mediated endocytosis, thereby forming an endosome containing the toxin. The toxin enters the cytoplasm of the cell by exiting the endosome. This step is considered to be mediated by HN, which is the amino-end fragment of the heavy chain causing the structural modification of the toxin at a pH of approximately 5.5 or less. The endosome is known to have a proton pump reducing the internal pH of the endosome. Due to structural displacement, hydrophobic residues of the toxin are exposed, allowing the toxin to be enclosed by the endosomal membrane. Afterward, the toxin (or the at least light chain) is translocated into cytosol through the endosomal membrane.

It is considered that the final step of the botulinum toxin activation mechanism includes the reduction of the disulfide bond connecting the heavy chain and the light chain. The total toxic activity of botulinum and tetanus toxins is included in the light chain of holotoxin; and the light chain is a zinc (Zn⁺⁺) endopeptidase selectively cleaving a protein that is necessary for recognition, docking of neurotransmitter-containing vesicles and the cytoplasmic surface of the plasma membrane, and fusion of the plasma membrane and the vesicles. Tetanus neurotoxin and botulinum toxin types B, D, F and G cause the degradation of synaptobrevin (also referred to as a vesicle-associated membrane protein (VAMP)), which is a synaptosomal membrane protein. VAMP present on the cytoplasmic surface of the synaptic vesicles is generally removed by a result of one of these degradation processes. Serotypes A and E cleave SNAP-25. Serotype C1 had been considered to cleave syntaxin, but was found to cleave syntaxin and SNAP-25. Except type B (and tetanus toxin) cleaving the same bond, each toxin specifically cleaves a different bond. Each of these cleavages interferes with the process of vesicle-membrane docking, preventing the exocytosis of the vesicle content.

Botulinum toxin is being used clinically for the treatment of neuromuscular disorders (i.e., movement disorders) characterized by hyperactive skeletal muscle. In 1989, the botulinum type A toxin complex was approved by the U.S. FDA to be used for treatment of essential blepharospasm, strabismus and hemifacial spasms. Subsequently, botulinum toxin type A toxin was also approved by the FDA to be used for treatment of cervical dystonia and glabellar lines, and botulinum toxin type B was also approved to be used in treatment of cervical dystonia. Botulinum serotypes, other than toxin type A, appear to have a lower potency and/or shorter duration in activity when compared with botulinum toxin type A. The clinical effect of botulinum toxin type A injected into a peripheral muscle is usually exhibited within a week after injection. The typical duration for symptom relief by a single intramuscular injection of botulinum toxin type A is approximately 3 months on average, but therapeutic activity for a significantly longer period was reported.

Although all botulinum toxin serotypes appear to inhibit the release of the neurotransmitter acetylcholine at neuromuscular junctions, they act on different neurosecretory proteins, and/or cleave these proteins at different sites. For example, both botulinum toxin types A and E cleave the 25-kD synaptosome-associated protein (SNAP-25), but target different amino acid sequences of the protein. Botulinum toxin types B, D, F and G act on VAMP (so-called synaptobrevin), and each serotype cleaves a different site of the protein. Finally, botulinum toxin type C1 appears to cleave both syntaxin and SNAP-25. The difference in these action mechanisms will affect the relative potency and/or duration of the actions of different botulinum toxin serotypes. Particularly, substrates for the botulinum toxin may be found in several different cell types.

The molecular weight of a botulinum toxin protein molecule is approximately 150 kD for all of the seven known botulinum toxin serotypes. Interestingly, by Clostridium bacteria, botulinum toxin is released as a complex containing the 150-kD botulinum toxin protein molecule along with an associated non-toxic protein. Therefore, the botulinum toxin type A complex may be produced by Clostridium bacteria as 900 kD, 500 kD and 300 kD types. Botulinum toxin types B and C1 appear to be produced only as a 700 kD or 500 kD complex. Botulinum toxin type D is produced as 300 kD and 500 kD complexes. Finally, botulinum toxin types E and F are produced only as an approximate 300 kD complex. These complexes (that is, those having larger molecular weights than approximately 150 kD) are considered to include a non-toxic hemagglutinin protein and a non-toxin, non-toxic, non-hemagglutinin protein. These two types of non-toxic proteins (including an associated neurotoxin complex as well as the botulinum toxin molecule) act to provide stability against the denaturation of the botulinum toxin molecule and protect the botulinum toxin molecule from digestive acids when the toxin is ingested. In addition, as the botulinum toxin complex is larger (molecule weight - approximately 150 kD or more), the botulinum toxin will more slowly diffuse from the site where the botulinum toxin complex was intramuscularly injected.

In addition, in *in vitro* research, it was shown that botulinum toxin inhibits potassium cation-induced release of acetylcholine and norepinephrine in the primary cell culture of brainstem tissue. In addition, it was reported that the botulinum toxin inhibits the induced release of glycine and glutamate in the primary culture of spinal cord neurons and the release of each of the neurotransmitters, acetylcholine, dopamine, norepinephrine, CGRP, substance P and glutamate, in brain synaptosome specimens. Accordingly, when used at an appropriate concentration, the botulinum toxin inhibits the stimulus-induced release of most neurotransmitters.

Botulinum toxin type A may be obtained by culturing a Clostridium Botulinum culture in a fermenter, and then harvesting and purifying the fermentation mixture by a known method. First, all of the botulinum toxin serotypes are synthesized as inactive single chain proteins, which have to be cleaved or nicked by a protease to be neuroactive. Bacterial strains producing botulinum toxin serotypes A and G have an endogenous protease, and thus the serotypes A and G may be usually recovered as an active form from the bacterial culture. In contrast, since botulinum toxin serotypes C1, D and E are synthesized by non-proteolytic strains, they are typically inactive when recovered from a culture. Since serotypes B and F may be produced by both proteolytic and non-proteolytic strains, they may be recovered as an active or inactive form. However, for example, a proteolytic strain producing botulinum toxin serotype B cleaves only a part of the produced toxin. The exact ratio of a nicked molecule to an unnicked molecule depends on a culture time and a temperature of the culture. Therefore, as previously known, since botulinum toxin type B has a significantly lower potency than botulinum toxin type A, for example, a certain percentage of the botulinum toxin type B of any specimen will be also inactive. The presence of an inactive botulinum toxin molecule in a clinical specimen will contribute to the overall protein load of the specimen and does not contribute to the clinical effect of the toxin, but is associated with an increase in antigenicity. In addition, at the same dose level, botulinum toxin type B is known to have a shorter activity duration and a lower potency when intramuscularly injected, compared to botulinum toxin type A.

High-quality crystalline botulinum toxin type A with a specific potency of ≥3 × 10⁷ U/mg, an A260/A278 of 0.60 or less, and having the characteristic of showing a distinct band pattern in gel electrophoresis may be obtained from the Hall A strain of *Clostridium Botulinum.* The known Shantz process may be used to obtain crystalline botulinum toxin type A. Generally, the botulinum toxin type A complex may be isolated and purified from an anaerobic fermentation obtained by culturing Clostridium Botulinum type A in a suitable medium. Such a known process may be used to isolate pure types of botulinum toxin from non-toxic proteins, for example, by purifying botulinum toxin type A with a molecular weight of approximately 150 kD and a specific potency of 1 to 2 × 10⁸ LD50 U/mg or more; purifying botulinum toxin type B with a molecular weight of approximately 156 kD and a specific potency of 1 to 2 × 10⁸ LD50 U/mg or more; or purifying botulinum toxin type F with a molecule weight of approximately 155 kD and a specific potency of 1 to 2 × 10⁷ LD50 U/mg or more.

Botulinum toxin and/or a botulinum toxin complex may be commercially available from a compound manufacturer known in the art, and pure botulinum toxin may also be used to prepare a pharmaceutical composition.

Generally, as in an enzyme, the biological activity of botulinum toxin (which is an intracellular peptidase) varies depending on at least in part, for example, the 3D structure thereof. Therefore, the toxicity of botulinum toxin type A may be removed by heat, various chemicals, surface scratches and surface drying. In addition, when a known toxin complex obtained by culture, fermentation and purification is diluted to a very low toxin concentration and used in the preparation of a pharmaceutical composition, when where is no appropriate stabilizer, it is known that the toxicity of the toxin is rapidly removed. Since specific toxicity is rapidly lost when the toxin complex is diluted in a large amount, it is considerably difficult to dilute several milligrams of the toxin in a solution containing several nanograms of the toxin per milliliter. Since the pharmaceutical composition containing the toxin may be prepared and then used for months or years, the toxin has to be stabilized using a suitable stabilizer. Therefore, as in the present invention, in order to stably control the release of botulinum toxin *in vivo,* it will be essential to develop optimal stabilizer technology.

The use of botulinum toxin type A in clinical settings was reported as follows:

The general duration of intramuscular injection in botulinum toxin administered once in vivo is typically approximately 3 to 4 months. However, in some cases, subtype A may have efficacy for 12 months or more, and when used to treat glands, such as in treatment of hyperhidrosis, in some circumstances, it may last for 27 months.

Botulinum toxin may not only exhibit a pharmacological action on the peripheral region, but may also exhibit an inhibitory effect on the central nervous system. It has been reported that botulinum toxin can be traced back to the spinal cord region by retrograde transport. As such, botulinum toxin administered to the peripheral site, for example, intramuscularly, may be transported retrogradely to the spinal cord.

Botulinum toxin has also been suggested or used to attempt to treat wounds in the skin, bones and tendons, pain, various autonomic nervous system disorders including hyperhidrosis, tension headaches, migraine pain, post-surgical pain and visceral pain, hair growth and hair maintenance, psoriasis and dermatitis, damaged muscles, various types of cancer, smooth muscle disorders, nerve entrapment syndrome, acne, neurogenic inflammation, ophthalmic diseases, pancreatic diseases, prostate diseases including benign prostatic hyperplasia, prostate cancer and incontinence, fibromyalgia, and piriformis syndrome.

It is known that modified Clostridial neurotoxin or a fragment thereof, preferably botulinum toxin, chemically conjugated or recombinantly fused to a specific targeting moiety, may be used to treat pain by administration to the spinal cord, and it is known that targeted botulinum toxin (i.e., having a non-native binding moiety) may be used to treat various diseases.

In addition, a technique for controlling pectoral spasms by injecting botulinum toxin into the pectoral muscle is also known, and as in the case of transdermal botulinum toxin administration, a controlled release toxin implant is known. It is known that botulinum toxin can be used to weaken chewing or biting muscles of the mouth so that self-inflicted wounds and resulting ulcers can be treated, treat benign gallbladder injuries or tumors, treat anal fissure, and treat certain types of atopic dermatitis.

In addition, botulinum toxin may have the effect of reducing inflammatory pain induced in a rat formalin model. Furthermore, it has been reported that botulinum toxin nerve blockage can cause a reduction in epidermal thickness. Finally, it is known to administer botulinum toxin to the foot to treat excessive foot sweating, spastic toes, idiopathic toe walking, and foot dystonia.

Tetanus toxin and derivatives (i.e., having a non-native targeting moiety), fragments, hybrids and chimeras thereof may also be used in treatment. Tetanus toxin is very similar to botulinum toxin. Thus, both tetanus toxin and botulinum toxin are polypeptides produced by closely related species of Clostridium *(Clostridium tetani* and *Clostridium botulinum,* respectively). In addition, both tetanus toxin and botulinum toxin are dichain proteins consisting of a light chain (molecular weight: approximately 50 kD) covalently binding to a heavy chain (molecular weight: approximately 100 kD) by a single disulfide bond. In addition, the molecular weight of tetanus toxin and each of the seven botulinum toxins (non-complexed) is approximately 150 kD. Moreover, in both the tetanus toxin and the botulinum toxin, the light chain includes a domain exhibiting intracellular biological (protease) activity, whereas the heavy chain includes receptor binding (immunogenic) and cell membrane translocation domains.

In addition, both the tetanus toxin and the botulinum toxin exhibit a high, specific affinity for gangliocide receptors on the surface of presynaptic cholinergic neurons. Retrograde axonal transport, blockage of the release of inhibitory neurotransmitter from central synapses, and spastic paralysis occur by receptor-mediated endocytosis of the tetanus toxin by peripheral cholinergic neurons. Conversely, the receptor-mediated endocytosis of the botulinum toxin by peripheral cholinergic neurons rarely results in retrograde transport, the inhibition of acetylcholine exocytosis from intoxicated peripheral motor neurons, and flaccid paralysis.

Finally, the tetanus toxin and the botulinum toxin resemble each other in both biosynthesis and molecular architecture. Accordingly, the tetanus toxin and botulinum toxin type A have an overall 34% identity in protein sequence, and 62% identity in sequence in some functional domains.

In one embodiment of the present invention, the "acetylcholine" is a neurotransmitter first discovered as an ester of choline and acetic acid, and distributed throughout the neuron. The formula of acetylcholine is C₇H₁₆NO₂, and its molecule weight is 146.21.

Although there is evidence suggesting that several neuromodulators can be released by the same neuron, typically, in the mammalian nervous system, only a single type of small molecule neurotransmitter is released by each type of neuron. The neurotransmitter acetylcholine is secreted by neurons in several regions of the brain, and particularly, the large pyramidal cells of the motor cortex, several different types of neurons in the basal ganglia, some types of motor neurons that are distributed in the skeletal muscles, preganglionic neurons of the autonomic nervous system (both sympathetic and parasympathetic nervous systems), bag 1 fiber of the muscle spindle fiber, the postganglionic neurons of the parasympathetic nervous system and the postganglionic neurons of the sympathetic nervous system. Originally, most of the postganglionic neurons of the sympathetic nervous system secrete the neurotransmitter norepinephrine, whereas only the postganglionic sympathetic nerve fibers to the sweat glands, the piloerector muscles and some blood vessels are cholinergic. In most cases, acetylcholine has an excitatory effect. However, acetylcholine is known to have inhibitory effects on some of the peripheral parasympathetic nerve endings (e.g., the inhibition of heart rate by the vagus nerve).

The efferent signals of the autonomic nervous system are transmitted to the body through either the sympathetic nervous system or the parasympathetic nervous system. The preganglionic neurons of the sympathetic nervous system extend from preganglionic sympathetic neuron cell bodies located in the intermediolateral horn of the spinal cord. The preganglionic sympathetic nerve fibers extending from the cell body form a synapse with postganglionic neurons located in either a paravertebral sympathetic ganglion or a prevertebral ganglion. Since the preganglionic neurons of the sympathetic and parasympathetic nervous systems are cholinergic, the application of acetylcholine to the ganglia may excite sympathetic and parasympathetic postganglionic neurons.

Acetylcholine activates two types of receptors, muscarinic and nicotinic receptors. The muscarinic receptors are found in all effector cells stimulated by the postganglionic neurons of the parasympathetic nervous system and the postganglionic cholinergic neurons of the sympathetic nervous system. Nicotinic receptors are found, as well as in the adrenal medulla, in the autonomic ganglia on the cell surface of the postganglionic neuron at the synapse between the preganglionic and postganglionic neurons of both the sympathetic nervous system and the parasympathetic nervous system. Nicotinic receptors are also found in many non-autonomic nerve endings, for example, in the membrane of skeletal muscle fibers at neuromuscular junctions.

Acetylcholine is released from cholinergic neurons when small, clear, intracellular vesicles are fused with the presynaptic neuronal cell membrane. A wide range of non-neuronal secretory cells such as the adrenal medulla (and the PC12 cell line) and pancreatic islet cells release catecholamines and parathyroid hormones, respectively, from large dense-core vesicles. The PC12 cell line is a clone of rat pheochromocytoma cells widely used as a tissue culture model for research on sympathoadrenal development. Botulinum toxin inhibits the release of two types of compounds from two types of cells *in vitro* when the toxin is allowed to permeate into denervated cells (by electroporation) or by direct injection of the toxin. Botulinum toxin is also known to inhibit the release of the neurotransmitter glutamate from a cortical synaptosome cell culture.

Neuromuscular junctions are formed in the skeletal muscle by exons around muscle cells. A signal transmitted through the nervous system results in an action potential at the terminal axon and activates ion channels, thereby releasing the neurotransmitter acetylcholine from synaptic vesicles in neurons at the motor endplate of the neuromuscular junction. The acetylcholine crosses the extracellular space and is bound with an acetylcholine receptor protein on the surface of the muscle endplate. Once the binding is sufficiently made, the action potential of the muscle cells causes the change in specific membrane ion channels, resulting in contraction of the muscle cells. Then, the acetylcholine is released from the muscle cells and metabolized by cholinesterase in the extracellular space. The metabolites are returned to the terminal axons for regenerating again into acetylcholine.

In one embodiment of the present invention, the "microstructure" includes microneedle, a microneedle, a microblade, a microknife, a microfiber, a microspike, a microprobe, a microbarb, a microarray or a microelectrode, but the present invention is not limited thereto. Particularly, in the present invention, the microstructure is preferably a microneedle. In addition, when a microneedle used for medical purposes as in the present invention is manufactured, the constituent is preferably a "biocompatible or biodegradable substance." Here, the "biocompatible substance" refers to a substance that is not toxic to a human body and chemically inactive. In addition, the "biodegradable substance" refers to a substance that can be degraded by body fluids, enzymes or microorganisms *in vivo.*

In the present invention, the microstructure is manufactured by including the botulinum toxin composition of the present invention, which includes botulinum toxin, a thickening agent and a stabilizer as active ingredients therein, or coated with the botulinum toxin composition of the present invention. Here, the microstructure includes any one that is manufactured by a microstructure manufacturing method used in the art, but the present invention is not limited thereto.

In one embodiment of the present invention, the "microneedle" refers to technology of increasing drug penetration by puncturing the skin with fine needles. The microneedle is used for drug delivery into the living body, blood collection, and detection of an analyte in the body.

The first known microstructure array was made of a silicon element using semiconductor process technology at the Georgia Institute of Technology in 1998 and presented the possibility as alternative technology for subcutaneous injection.

Unlike other existing needles, the microneedles are characterized by painless skin penetration and no trauma, and for the painless skin penetration, the upper end diameter for minimal invasiveness is important. In addition, since the microneedles have to penetrate the 10 to 20-µm stratum corneum, which is the strongest barrier of the skin, it is required to have a sufficient physical hardness. In addition, the appropriate length for increasing the efficiency of drug delivery by reaching the capillaries has to be considered, too.

After in-plane type microneedles have been previously suggested, various types of microneedles were developed. According to a method of manufacturing out-of-plane type solid microneedles using an etching method, solid silicon microneedles having a diameter of 50 to 100 µm and a diameter of 500 µm were manufactured, but it was impossible to implement painless skin penetration, and it was difficult to deliver a drug and a cosmetic ingredient to a desired area.

Meanwhile, Prausnitz of the University of Georgia, USA, has proposed a method of manufacturing biodegradable polymer microneedles by etching glass or forming a mold through photolithography. In addition, a method of manufacturing biodegradable solid microneedles by loading a substance prepared in the form of a capsule at the end of the mold manufactured through photolithography was proposed in 2006. According to this method, while there is an advantage of being free to load drugs that can be manufactured in the form of a capsule, as the drug load increases, the hardness of the microneedle weakens, so there is a limit to application to a drug requiring large-scale administration.

In 2005, absorptive microneedles were proposed by Nano Devices & Systems. Such absorptive microneedles are to be used in drug delivery or cosmetics without removing the microneedles inserted into the skin. According to this method, microneedles were manufactured by applying a composition prepared by mixing maltose with a drug to a mold and solidifying the composition. The Japanese Patent proposes transdermal absorption of a drug by manufacturing microneedles in an absorptive type, but these microneedles accompanied pain while skin penetration. In addition, due to the technical limitation of mold manufacturing, it was impossible to manufacture microneedles having a suitable upper end diameter causing no pain, and a length required for effective drug delivery, that is, 1 mm or more.

In 2008, biodegradable microneedles manufactured by Prausnitz at the University of Georgia, USA were manufactured using a substance which is a mixture of polyvinylpyrrolidone (PVP) and methacrylic acid (MAA) in a polydimethylsiloxane (PDMS) mold. In addition, microneedles were also manufactured by putting carboxymethyl cellulose into a pyramid-shaped mold. However, the manufacturing method using a mold has a limitation in the formation of a new mold through complicated procedures in order to adjust the diameter and length of microneedles, and disadvantages in that the process of manufacturing microneedles by adding a substance into a mold is complicated and takes a long time.

In 2008, the equipment and method for manufacturing skin needles using a pin structure were reported. The method is for pulling a base on a substrate with pins by heating or using the tensile force of a viscous material. Since this method uses a method of pulling a material melted or viscous by heat with a pin structure, it cannot overcome the limitations that production costs increase due to the necessity of a procedure of newly forming a pin structure according to a desired pattern, and it is difficult to load various biomedicines sensitive to heat (e.g., hormones, vaccines, and other protein drugs) due to a heating process.

Meanwhile, the skin consists of the stratum corneum (< 20 µm), the epidermis (< 100 µm), and the dermis (300 to 2,500 µm) from the surface. Therefore, in order to deliver drugs and physiologically active substances to a specific skin layer without pain, it is effective for delivery of drugs and cosmetic ingredients that microneedles are manufactured to have an upper end diameter of approximately 30 µm, an effective length of 200 to 2,000 µm, and a sufficient hardness for skin penetration. In addition, in order to deliver drugs and physiologically active materials through biodegradable solid microneedles, it is necessary to exclude a process that can destroy the activity of drugs and physiologically active substances, for example, high heat treatment or organic solvent treatment, in the microneedle manufacturing process.

Conventional solid microneedles were limited to materials such as silicon, polymers, metals and glass due to the limitation in the manufacturing method, and had disadvantages of drug degeneration, insufficient hardness, and drug loss due to complicated and long-term production by using a manufacturing method through molding technology. Therefore, the demand for formulation technology for microneedles that have a diameter small enough to cause no pain in skin penetration, a sufficiently long length to deeply penetrate into the skin, have a sufficient hardness without particular limitation to a material, and minimize drug loss is continuing.

In the present invention, the microstructure is characterized by being coated with a composition including botulinum toxin, and to improve coating stability and efficiency, may be pretreated with a polymer compound. When the microstructure is a microneedle, the polymer compound is preferably a polylactic acid (PLLA) material, and the polymer compound for pretreatment is preferably polyvinyl alcohol (PVA), but the present invention is not limited thereto. The concentration of PVA is preferably 1 to 10%, more preferably 1 to 5%, even more preferably 1 to 3%, and even more preferably 2%.

In one embodiment of the present invention, the "pharmaceutical composition" refers to a composition administered for a specific purpose. For the purpose of the present invention, the pharmaceutical composition of the present invention is to inject the botulinum toxin whose concentration is finely adjusted at the exact administration site, and may include a protein involved therein and a pharmaceutically acceptable carrier, an excipient or a diluent. The "pharmaceutically acceptable" carrier or excipient refers to one that has been approved by the regulatory department of the government or is listed in the pharmacopeia approved by the government or other generally approved pharmacopeia for use in vertebrates, and more particularly in humans.

The microstructure including botulinum toxin suitable for parenteral administration as an active ingredient may be prepared in a suspension, solution or emulsion in an oily or aqueous carrier, or in solid or semi-solid form and may include a formulatory agent(s) such as a suspending agent, a stabilizer, a dissolving agent and/or a dispersant. This form may be sterilized, and may be a liquid. It may be stabilized under preparation and storage conditions, and preserved against the contamination by microorganisms such as bacteria or fungi. Alternatively, the microstructure including botulinum toxin as an active ingredient may be a sterilized powder for reconstitution with a suitable carrier before use. The pharmaceutical composition may be present in a unit-dose form, in a microneedle patch, in ampoules, or in other unit-dose or multi-dose containers. Alternatively, the pharmaceutical composition may be stored in a freeze-dried (lyophilized) state requiring the addition of a sterile liquid carrier, for example, water for injection immediately before use. Immediate injection solutions and suspensions may be prepared as a sterile powder, granule or tablet.

In some non-limiting embodiments, the microstructure including botulinum toxin as an active ingredient may be formulated in a liquid, or may be included in the form of microspheres. In a non-limiting embodiment, a microstructure liquid composition including botulinum toxin as an active ingredient includes botulinum toxin, or a pharmaceutically acceptable compound and/or mixture thereof at 0.001 to 100,000 U/kg. In addition, in a non-limiting embodiment, a suitable excipient for the microstructure composition including botulinum toxin as an active ingredient includes a preservative, a suspending agent, a stabilizer, a dye, a buffer, an antibacterial agent, an antifungal agent, and an isotonic agent, for example, sugar or sodium chloride. Here, the term "stabilizer" used herein refers to a compound selectively used in the pharmaceutical composition of the present invention to increase the lifetime thereof. In a non-limiting embodiment, the stabilizer may be a saccharide, an amino acid, a compound, or a polymer, wherein the saccharide may be a monosaccharide, a disaccharide, or a polysaccharide, and preferably, a disaccharide. The disaccharide is classified into a reducing disaccharide, and a non-reducing disaccharide, wherein the reducing disaccharide has a free "hemiacetal" unit in which one monosaccharide of the two "reducing sugars" can act as a reducing "aldehyde," and the non-reducing disaccharide is produced by the acetal-forming bond between anomeric carbons of two monosaccharides, so there is no hemiacetal that can serve as a reducing agent. Examples of reducing disaccharides include cellobiose (Cel) and maltose (Mal), and examples of the non-reducing disaccharides include trehalose (Tre) and sucrose (Suc). The pharmaceutical composition may include one or more pharmaceutically acceptable carriers. The carrier may be a solvent or a dispersion medium. Non-limiting examples of the pharmaceutically acceptable carriers include water, physiological saline, ethanol, polyols (e. g., glycerol, propylene glycol and liquid polyethylene glycol), oils, an appropriate mixture thereof.

A parenteral formulation may be sterilized. Non-limiting examples of sterilizing techniques include filtration through a bacteriostatic filter, terminal sterilization, incorporation of sterile agents, irradiation, application of sterile gas, heating, vacuum drying and freeze drying.

In one embodiment of the present invention, the "administration" refers to introduction of the composition of the present invention to a patient by an appropriate method, and the administration route of the composition of the present invention may be any general route whereby the composition of the present invention can reach target tissue. The administration route may be oral administration, intraperitoneal administration, intravenous administration, intramuscular administration, subcutaneous administration, intradermal administration, intranasal administration, intrapulmonary administration, intrarectal administration, intracavitary administration, or intrathecal administration, but in the case of a microstructure including the botulinum toxin composition of the present invention as an active ingredient, intradermal administration using microneedles is preferable.

The treatment method of the present invention may include administering the pharmaceutical composition in a pharmaceutically effective amount. The effective amount of the present invention may be adjusted according to various factors including the type or severity of a disease, the types and contents of the active ingredient and other ingredients contained in the composition, the type of formulation, a patient's age, weight, general health condition, gender and diet, administration time, an administration route, the release rate of the composition, treatment duration, and a concurrently used drug.

In one embodiment of the present invention, a botulinum toxin composition including botulinum toxin, a thickening agent and a stabilizer as active ingredients is provided, wherein the botulinum toxin is one or more selected from the group consisting of botulinum toxin types A, B, C, D, E, F and G, the thickening agent is one or more selected from the group consisting of carboxymethyl cellulose, sodium salt; sodium alginate; hyaluronic acid; methyl cellulose; hydroxyethyl cellulose; and polyvinyl pyrrolidone, and is included at 0.05 to 10 wt%, and the stabilizer is one or more selected from the group consisting of trehalose, sucrose, a mixture of trehalose and sucrose, methionine, sodium phosphate, and a mixture of human serum albumin and sodium chloride, and is included at 0.03 to 50 wt%. As the stabilizer, the mixture of trehalose and sucrose is a mixture of approximately 5, 10, 20, 30, 40, 50, 60, 70, 80, 90 or 95% trehalose and approximately 95, 90, 80, 70, 60, 50, 40, 30, 20, 10 or 5% sucrose.

In another embodiment of the present invention, a microstructure including the botulinum toxin composition is provided, and is one or more selected from the group consisting of a microneedle, a microblade, a microknife, a microfiber, a microspike, a microprobe, a microbarb, a microarray, and a microelectrode.

In another embodiment of the present invention, a method of preparing a botulinum toxin composition, which includes mixing botulinum toxin, a thickening agent and a stabilizer, is provided, wherein the botulinum toxin is one or more selected from the group consisting of botulinum toxin types A, B, C, D, E, F and G, the thickening agent is one or more selected from the group consisting of carboxymethyl cellulose, sodium salt; sodium alginate; hyaluronic acid; methyl cellulose; hydroxyethyl cellulose; and polyvinyl pyrrolidone, and mixed at 0.05 to 10 wt%, and the stabilizer is one or more selected from the group consisting of trehalose, sucrose, a mixture of trehalose and sucrose, methionine, sodium phosphate, and a mixture of human serum albumin and sodium chloride, and is mixed at 0.03 to 50 wt%.

In still another embodiment of the present invention, a method of coating a target object with a botulinum toxin composition, which includes (a) pretreatment coating a target object with a polymer compound; (b) mixing botulinum toxin, a thickening agent and a stabilizer; and (c) adding the mixture of (b) to the pretreatment-coated subject of (a) dropwise, followed by drying, is provided. In this method, the polymer compound is a polyvinyl alcohol, which is used at 0.5 to 10 wt%, the botulinum toxin is botulinum toxin type A, the thickening agent is one or more selected from the group consisting of carboxymethyl cellulose, sodium salt; sodium alginate; hyaluronic acid; methyl cellulose; hydroxyethyl cellulose; and polyvinyl pyrrolidone, the stabilizer is one or more selected from the group consisting of trehalose, and/or sucrose, a mixture of trehalose and sucrose, methionine, sodium phosphate, and a mixture of human serum albumin and sodium chloride, and the target object is a microstructure, which is microneedles.

In yet another embodiment of the present invention, a composition for coating a microstructure, which includes botulinum toxin, hydroxyethyl cellulose and sucrose, is provided, wherein the botulinum toxin is one or more selected from the group consisting of botulinum toxin types A, B, C, D, E, F, and G, the microstructure is one or more selected from the group consisting of a microneedle, a microblade, a microknife, a microfiber, a microspike, a microprobe, a microbarb, a microarray, and a microelectrode.

In the composition for coating a microstructure, the mixing ratio (w:w) of hydroxyethyl cellulose and sucrose may be more than 30 to less than 90 of sucrose, 50 to 80, 60 to 80, or 60 to 70, with respect to 1 of hydroxyethyl cellulose.

In the composition for coating a microstructure, the mixing ratio (w:w) of hydroxyethyl cellulose and sucrose may be more than 30 to less than 90 of sucrose, preferably 50 to 80, more preferably 60 to 80, and even more preferably 60 to 70, with respect to 1 of hydroxyethyl cellulose.

In yet another embodiment of the present invention, a microstructure coated with the composition for coating a microstructure is provided. In this method, the microstructure is one or more selected from the group consisting of a microneedle, a microblade, a microknife, a microfiber, a microspike, a microprobe, a microbarb, a microarray, and a microelectrode.

In yet another embodiment of the present invention, a method of preparing a composition for coating a microstructure, which includes mixing botulinum toxin, hydroxyethyl cellulose and sucrose, is provided. In this method, the botulinum toxin is one or more selected from the group consisting of botulinum toxin types A, B, C, D, E, F, and G, the microstructure is one or more selected from the group consisting of a microneedle, a microblade, a microknife, a microfiber, a microspike, a microprobe, a microbarb, a microarray, and a microelectrode.

In yet another embodiment of the present invention, a method of coating a microstructure with a composition including botulinum toxin, which includes (a) mixing botulinum toxin, hydroxyethyl cellulose, and sucrose; and (b) coating a microstructure surface with the mixture of (a), is provided. In this method, the botulinum toxin is one or more selected from botulinum toxin types A, B, C, D, E, F, and G, and the microstructure is one or more selected from the group consisting of a microneedle, a microblade, a microknife, a microfiber, a microspike, a microprobe, a microbarb, a microarray, and a microelectrode.

Hereinafter, the present invention will be described in detail step by step.

### [Advantageous Effects]

Botulinum toxin causes systemic asthenia by inhibiting acetylcholine exocytosis at the cholinergic presynapse of neuromuscular junctions in animals with nerve function. The neurotoxic function of botulinum toxin has high toxicity and thus is fatal in a very small amount, when the toxin is used for a living body, technology for fine concentration control and technology for administration to an exact location are essentially required. The present invention relates to a microstructure formulation technique for botulinum toxin, which uses a microstructure of the present invention to reduce pain during the administration of botulinum toxin and facilitate the administration of a small amount of toxin to an exact location. Therefore, the present invention is expected to be broadly applied for safe and convenient medical use.

### [Description of Drawings]

FIG. 1 shows a recovery rate with respect to the absolute potency of botulinum toxin according to a pretreatment coating material according to one embodiment of the present invention.

FIG. 2 shows a recovery rate with respect to the absolute potency of botulinum toxin according to the type and concentration of a thickening agent mixed with botulinum toxin according to one embodiment of the present invention.

FIG. 3 shows a recovery rate with respect to the absolute potency of botulinum toxin according to the type and concentration of a stabilizer mixed with botulinum toxin according to one embodiment of the present invention.

FIG. 4 shows a result of long-term stability of the combination of a SA or HA thickening agent with a stabilizer according to one embodiment of the present invention.

FIG. 5 shows a result of long-term stability of the combination of a PVP or HEC thickening agent and a stabilizer according to one embodiment of the present invention.

FIG. 6 shows a result of confirming the coating uniformity of a coating composition including botulinum toxin according to one embodiment of the present invention.

FIG. 7 shows a result of regression analysis of measured viscosity values of a coating composition by mixing ratio of a HEC:Suc combination according to one embodiment of the present invention.

FIG. 8 shows a result of regression analysis of measured surface tension values of a coating composition by mixing ratio of a HEC:Suc combination according to one embodiment of the present invention.

FIG. 9 is a set of images showing the result of uniformity of a coating composition by mixing ratio of a HEC:Suc combination according to one embodiment of the present invention.

FIG. 10 shows a result of coating uniformity of a coating composition as a result of regression analysis for relative standard deviations of the total pixel number by mixing ratio of a HEC:Suc combination according to one embodiment of the present invention.

FIG. 11 shows a result of long-term stability of a coating composition by mixing ratio of a HEC:Suc combination according to one embodiment of the present invention.

### [Modes of the Invention]

To test the long-term stability of the combination of a PVP or HEC thickening agent, and a disaccharide or amino acid stabilizer, an accelerated stability test was carried out at 37 °C for 5 weeks after the preparation and dropwise-addition of a botulinum toxin-coating composition. As a disaccharide stabilizer, trehalose (Tre) and/or sucrose (Suc) were(was) used, and as an amino acid stabilizer, methionine (Met) and glycine (Gly) were used. As a result, it was confirmed that a condition using PVP or HEC as a thickening agent and a disaccharide or methionine as a stabilizer is the optimal stabilized combination for preparing a botulinum toxin-coating composition.

### [Examples]

Hereinafter, the present invention will be described in further detail with reference to examples. The examples are merely provided to more specifically explain the present invention, and it will be obvious to those of ordinary skill in the art that the scope of the present invention is not limited to the examples according to the gist of the present invention.

### Example 1. Confirmation of dilution factor for botulinum toxin potency test

Since the potency loss of botulinum toxin, occurring when a 2X coating composition and a 2X botulinum toxin composition were mixed, is unknown, a preliminary test was carried out to confirm the change in potency immediately after the preparation of the botulinum toxin composition.

To this end, the 2X coating composition and the 2X botulinum toxin composition were separately prepared, and mixed at 1:1, thereby preparing a mixed composition as shown in Table 1.

**[Table 1]**

| Preparation Example | Concentration after final mixing of 2X coating composition and 2X botulinum toxin composition |
|---|---|
| HSA+NaCl | 0.5% HSA, 0.9% NaCl, 5,000 U Toxin/6.5 *µ*ℓ |
| Tween20 | 0.2% Tween20, 5,000 U Toxin/6.5 *µ*ℓ |
| CMC | 1% CMC, 5,000 U Toxin/6.5 *µ*ℓ |
| Sodium phosphate (pH6.0) | 0.025 M Sodium phosphate, 5,000 U Toxin/6.5 *µ*ℓ |
| HSA (Human serum albumin) | |
| NaCl (Sodium chloride) | |
| CMC(CarboxyMethylCellulose sodium salt) | |

For the mixed composition, the potency of botulinum toxin was detected using an *in vitro* potency measuring kit (BoTest^{®} Matrix Botulinum Neurotoxin Detection Kits, Biosentinel, Inc). A detailed method was conducted in the same manner as disclosed in a prior document written by Dunning FM (J Vis Exp. 2014 Mar 3;(85). doi: 10.3791/51170.). In addition, two dilution factors (75 and 112.5) were applied in case of potency loss immediately after preparation. The result is shown in Table 2.

**[Table 2]**

| Preparation Example | Estimated potency (U/6.25 *µ*ℓ) | Dilution factor | Potency (U) | Average | SD | RSD | Recovery rate [%] |
|---|---|---|---|---|---|---|---|
| HSA+NaCl | 5000 | 75 | 6169.0 | 6240 | 100.7 | 1.6 | 124.8 |
| | | 112.5 | 6311.4 | | | | |
| Tween20 | 5000 | 75 | 6465.4 | 6561 | 135.2 | 2.1 | 131.2 |
| | | 112.5 | 6656.7 | | | | |
| CMC | 5000 | 75 | 3235.5 | 3427 | 270.9 | 7.9 | 68.5 |
| | | 112.5 | 3618.6 | | | | |
| Sodium phosphate (pH6.0) | 5000 | 75 | 4275.5 | 4423 | 208.7 | 4.7 | 88.5 |
| | | 112.5 | 4570.7 | | | | |

As a result of measuring the potency of botulinum toxin with respect to the mixed composition, it was confirmed that 50% or more of the absolute potency was recovered. However, in the case of CMC, when a dilution factor of 112.5 was applied, the potency was out of the range of a standard curve, and the potencies in the cases of CMC and sodium phosphate were lower than those of HSA or Tween20 immediately after preparation.

### Example 2. Selection of pretreatment coating material

This example is to confirm the change in potency of botulinum toxin according to the presence or absence of a pretreatment coating of a poly(lactic acid) (PLA) substrate.

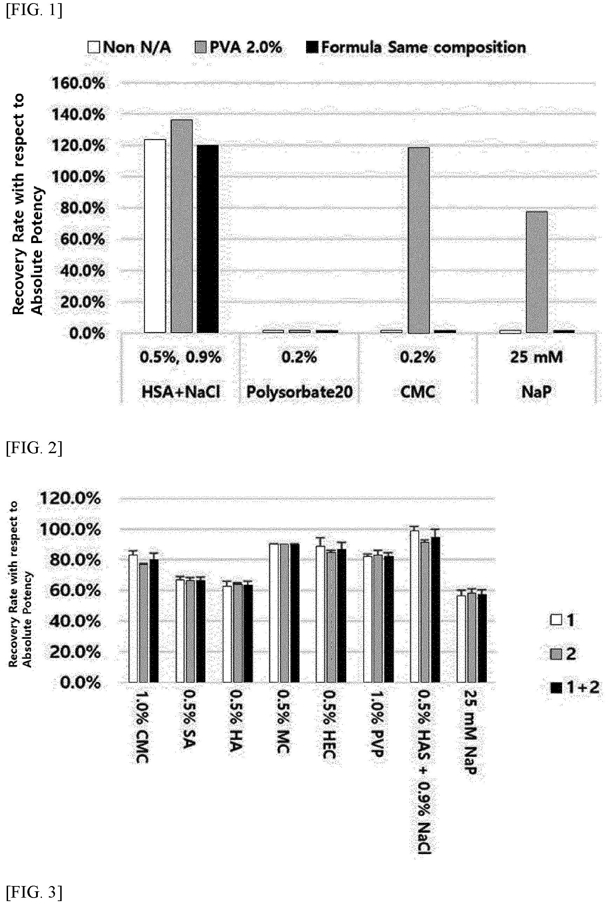

6.5 µl each of coating compositions of HSA+NaCl, Tween20, CMC, and sodium phosphate (pH 6.0), which were mixed with botulinum toxin was added dropwise on a PLA substrate (non-coating (N), 2% PVA pretreatment coating (PVA), or a corresponding thickening agent (e.g., when 1% HSA+1.8% NaCl was used as a thickening agent, the PLA substrate was also coated with 1% HSA + 1.8% NaCl)), and dried at room temperature overnight (O/N). After drying, each substrate was put in 0.3 ml of 0.9% NaCl contained in a 1.5 ml tube to elute the toxin at room temperature for 30 minutes. After the elution was completed, an *in vitro* potency assay was carried out by dilution with a dilution factor of 83.3, assuming that the botulinum toxin potency was preserved 100% during recovery after dropwise addition. The result is shown in Table 3 and FIG. 1.

**[Table 3]**

| Preparation Example | Substrate Pretreatment coating | Estimated potency (U) | Dilution factor | Potency (U) | Recovery rate[%] |
|---|---|---|---|---|---|
| HSA+NaCl | N | 5000 | 83.3 | 6195.2 | 123.9 |
| | PVA | 5000 | 83.3 | 6811.5 | 136.2 |
| | Corresponding thickening agent | 5000 | 83.3 | 6020.7 | 120.4 |
| Tween20 | N | 5000 | 83.3 | N/D | low |
| | PVA | 5000 | 83.3 | N/D | low |
| | Corresponding thickening agent | 5000 | 83.3 | N/D | low |
| CMC | N | 5000 | 83.3 | N/D | low |
| | PVA | 5000 | 83.3 | 5950.5 | 119.0 |
| | Corresponding thickening agent | 5000 | 83.3 | N/D | low |
| Sodium phosphate (pH6.0) | N | 5000 | 83.3 | N/D | low |
| | PVA | 5000 | 83.3 | 3883.3 | 77.7 |

As shown in the above result, in the process of recovery after dropwise addition, when the potency exceeds the range of the standard curve, it is represented as N/D. Considering the sodium phosphate result, it was found that the potency was stably retained when the pretreatment coating on the substrate was performed with 2% PVA.

It was determined that Tween20 is not appropriate as a coating composition because low potency was shown from all substrates, and in the case of CMC, like the case of sodium phosphate, potency was not able to be measured from all substrates except the the substrate subjected to pretreatment coating with PVA.

### Example 3. Selection of thickening agent optimized for coating composition including botulinum toxin

To select a thickening agent to be included in the coating composition among candidate materials, each of 8 types of candidate coating materials was prepared at a 2X concentration (2% CMC, 1% SA, 1% HA, 1% MC, 1% HEC, 2% PVP, 1% HSA+1.8% NaCl, and 0.05M phosphate), and mixed with a 2X botulinum toxin solution (5,000 U/3.25 µl), thereby preparing a 1:1 solution. The resulting solution was added dropwise at 6.5 µl on a substrate subjected to pretreatment coating with 2% PVA, followed by drying the resulting substrate overnight (O/N) at room temperature. After drying was completed, each substrate was put in 0.3 ml of 0.9% NaCl contained in a 1.5 ml tube, and the toxin was eluted at room temperature for 30 minutes. After the elution was completed, an *in vitro* potency assay was carried out by dilution with a dilution factor of 83.3, assuming that the potency was preserved 100% during recovery after dropwise addition. The result is shown in Table 4 and FIG. 2.

**[Table 4]**

| Type of coating solution | n | Potency (U) | Total Average | Total SD | Total RSD | Total average recovery rate [%] |
|---|---|---|---|---|---|---|
| 1. CMC | 1-1 | 4232.3 | 4004.4 | 177.7 | 4.4 | 80.1 |
| | 1-2 | 4057.9 | | | | |
| | 2-1 | 3877.9 | | | | |
| | 2-2 | 3849.6 | | | | |
| 2. SA | 1-1 | 3412.6 | 3356.6 | 51.4 | 1.5 | 67.1 |
| | 1-2 | 3383.3 | | | | |
| | 2-1 | 3381.0 | | | | |
| | 2-2 | 3294.6 | | | | |
| 3. HA | 1-1 | 3216.0 | 3193.5 | 72.5 | 2.3 | 63.9 |
| | 1-2 | 3086.3 | | | | |
| | 2-1 | 3245.7 | | | | |
| | 2-2 | 3226.1 | | | | |
| 4. MC | 1-1 | 4494.5 | 4507.2 | 10.1 | 0.2 | 90.1 |
| | 1-2 | 4518.9 | | | | |
| | 2-1 | 4509.8 | | | | |
| | 2-2 | 4505.7 | | | | |
| 5. HEC | 1-1 | 4627.1 | 4356.6 | 183.2 | 4.2 | 87.1 |
| | 1-2 | 4286.5 | | | | |
| | 2-1 | 4221.3 | | | | |
| | 2-2 | 4291.4 | | | | |
| 6. PVP | 1-1 | 4097.1 | 4137.5 | 83.1 | 2.0 | 82.8 |
| | 1-2 | 4165.6 | | | | |
| | 2-1 | 4048.2 | | | | |
| | 2-2 | 2439.2 | | | | |
| 7. Sodium phospate | 1-1 | 2915.0 | 2891.0 | 81.4 | 2.8 | 57.8 |
| | 1-1 | 2787.1 | | | | |
| | 2-1 | 2982.6 | | | | |
| | 2-1 | 2879.5 | | | | |
| 8. HSA + NaCl | 1-1 | 5044.6 | 4769.7 | 216.7 | 4.5 | 95.4 |
| | 1-2 | 4837.7 | | | | |
| | 2-1 | 4567.2 | | | | |
| | 2-2 | 4629.2 | | | | |
| CMC (CarboxyMethylCellulose sodiu salt) | | | | | | |
| SA (Sodium Alignate) | | | | | | |
| HA (Hyaluronic acid 1,000kDa) | | | | | | |
| MC (Methyl cellulose) | | | | | | |
| HEC (HydroxyEthyl-Cellulose) | | | | | | |
| PVP (PolyVinylPyrrolidone) | | | | | | |

As shown in Table 4, first, as a result of preparing two samples (samples 1 and 2) in each experimental group and measuring the toxin eluted from each sample twice, the recovery rates of CMC, MC, HEC, PVP and HSA+NaCl of the coating compositions were as high as 80% or more with respect to the target potency, and particularly, the recovery rates of MC and HSA+NaCl were 90% or more. In addition, it was confirmed that the average recovery rates of SA and HA were 60% or more, which were higher than that of sodium phosphate.

### Example 4. Selection of stabilizer optimized for coating composition including botulinum toxin

To select a stabilizer included in the coating composition among candidate materials, as shown in Table 5, a 1:1 solution prepared by mixing a 2X botulinum toxin solution (5,000 U/3.25 µl) with each of the coating compositions prepared as shown in Table 5 was added dropwise at 6.5 µl on the substrate subjected to pretreatment coating with 2% PVA, and dried overnight (O/N) at room temperature. After drying was completed, each substrate was put in 0.3 ml of 0.9% NaCl contained in a 1.5 ml tube, and the toxin was eluted at room temperature for 30 minutes. After the elution was completed, an *in vitro* potency assay was carried out by dilution with a dilution factor of 83.3, assuming that the potency was preserved 100% during recovery after dropwise addition.

**[Table 5]**

| Preparation Example | 2X concentration |
|---|---|
| 1. PVP+Tre 10% | 2% PVP. 20% Tre |
| 2. PVP+Tre 15% | 2% PVP, 30% Tre |
| 3. PVP+Tre 20% | 2% PVP, 40% Tre |
| 4. PVP+Met 10mM | 2% PVP, 20mM Met |
| 5. PVP+Met 20mM | 2% PVP, 40mM Met |
| 6. PVP+Met 30mM | 2% PVP, 60mM Met |
| 7. HEC+Tre 10% | 1% HEC, 20% Tre |
| 8. HEC+Tre 15% | 1% HEC, 30% Tre |
| 9. HEC+Tre 20% | 1% HEC, 40% Tre |
| 10. HEC+Met 10mM | 1% HEC, 20mM Met |
| 11. HEC+Met 20mM | 1% HEC, 40mM Met |
| 12. HEC+Met 30mM | 1% HEC, 60mM Met |
| 13. CMC+Tre 10% | 2% CMC, 20% Tre |
| 14. CMC+Tre 15% | 2% CMC, 30% Tre |
| 15.CMC+Tre 20% | 2% CMC, 40% Tre |
| 16. CMC+Met 10mM | 2% CMC, 20mM Met |
| 17. CMC+Met 20mM | 2% CMC, 40mM Met |
| 18. CMC+Met 30mM | 2% CMC, 60mM Met |
| 19. HSA+NaCl | 1% HSA, 1.8% NaCl |
| 20. Sodium phosphate | 0.05M Sodium phosphate |
| Tre (Trehalose) | |
| Met (Methionine) | |

According to the experiment result, it was confirmed that the PVP and HEC groups except for CMC showed a recovery rate of 90% or more compared to that of immediately after HSA preparation in all stabilizer mixing conditions. The result is shown in FIG. 3.

### Example 5. Confirmation of long-term stability of coating composition including botulinum toxin

The long-term stability of mixtures of the thickening agents (SA, HA, PVP, and HEC) selected from the results of Examples 1 to 4 and stabilizers (Tre and Met) was confirmed

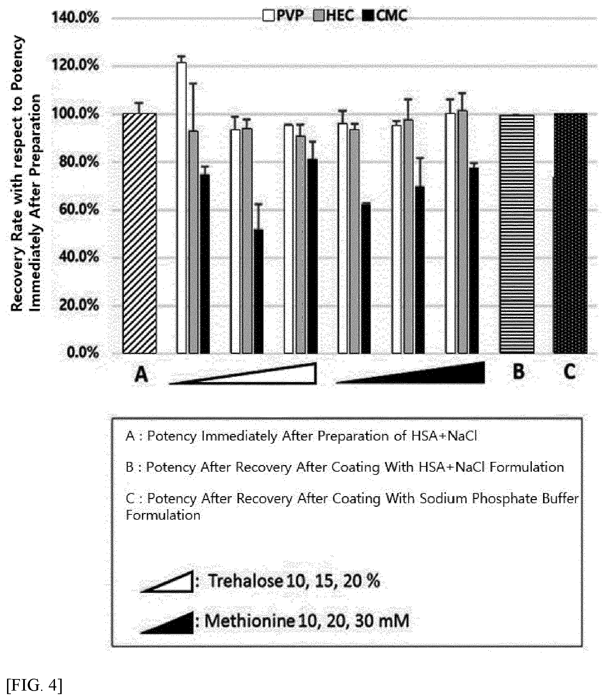

First, to test the long-term stability of the combinations of the SA and HA thickening agents and the stabilizers, a PLA substrate was subjected to pretreatment coating with 2% PVA, and a 1:1 mixture of a 2X botulinum toxin solution (5,000 U/3.25 µl) and each of the coating compositions prepared as shown in Table 6 was added dropwise at 6.5 µl, followed by drying the resulting substrate overnight (O/N) at room temperature. After drying was completed, each substrate was put in 0.3 ml of 0.9% NaCl contained in a 1.5 ml tube to measure a potency recovery rate until day 8. Since the coating compositions in which the thickening agents and the stabilizers in various combinations can affect the measurement of the potency of the botulinum toxin, the potency measured on day 1 in each experimental group was used as the reference value (100), and the potency measured on day 8 was converted based on the potency on day 1, and is shown in FIG. 4. According to the experiment result, in most of the experimental groups using the SA and HA thickening agents, it was confirmed that the potency was retained 50% or more compared to the potency on day 1 until day 8.

**[Table 6]**

| Preparation Example | Note |
|---|---|
| 1. SA+Tre 10% | 0.5% Sodium Alginate, 10% Trehalose |
| 2. SA+Tre 15% | 0.5% Sodium Alginate, 15% Trehalose |
| 3. HA+Tre 10% | 0.5% Hyaluronic acid, 10% Trehalose |
| 4. HA+Tre 15% | 0.5% Hyaluronic acid, 15% Trehalose |
| 5. HA+Tre 20% | 0.5% Hyaluronic acid, 20% Trehalose |
| 6. HA+Met 10 mM | 0.5% Hyaluronic acid, 10 mM Methionine |
| 7. HA+Met 20 mM | 0.5% Hyaluronic acid, 20 mM Methionine |
| 8. HA+Met 30 mM | 0.5% Hyaluronic acid, 30 mM Methionine |
| 9. PVP+Tre 10% | 1% PolyVinylPyrrolidone, 10% Trehalose |
| 10. PVP+Met 20 mM | 1% PolyVinylPyrrolidone, 20 mM Methionine |
| 11. HAS+NaCl | 0.5% Human Serum Albumin, 0.9% Sodium Chloride |
| 12. NaP | 25mM Sodium Phosphate pH 6.0 |

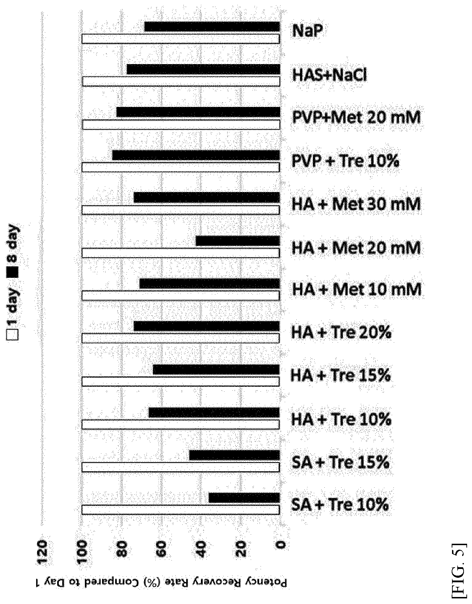

In addition, to test the long-term stability of the combination of the PVP and HEC thickening agents and a disaccharide or amino acid stabilizer, botulinum toxin-coating compositions were prepared in the same manner as in the SA and HA tests, and each of the compositions was added dropwise, followed by performing an accelerated stability test at 37 °C for 5 weeks. Trehalose (Tre) and/or sucrose (Suc), as a disaccharide stabilizer(s), and methionine (Met) and glycine (Gly), as amino acid stabilizers, were used. For accurate comparison of experiment values, the potency immediately after the preparation of each sample was converted to 100%, and potency recovery rates for 5 weeks were compared. In addition, errors between tests were minimized by applying the value of the standard preparation (BoTest^{®}), which is a positive control. The result is shown in Table 7 and FIG. 5.

**[Table 7]**

| Preparation Example | Immediately after preparation | 1day | 8day | 22day | 36day |
|---|---|---|---|---|---|
| 1% PVP | 100.0 | 92.3 | 71.2 | 61.1 | N/D |
| 1% PVP + 10% Tre | 100.0 | 107.9 | 85.1 | 70.2 | 57.2 |
| 1% PVP + 30% Suc | 100 | 239.5 | 154.7 | 154.7 | 68.1 |
| 1% PVP + Met 20mM | 100.0 | 108.4 | 105.9 | 87.7 | 55.0 |
| 1 % PVP + Gly 20mM | 100 | 79.1 | N/D | N/D | N/D |
| 0.5% HEC | 100.0 | 80.7 | 65.2 | N/D | N/D |
| 0.5% HEC + 10% Tre | 100.0 | 92.5 | 88.9 | 66.0 | 53.4 |
| 0.5% HEC + 30% Sue | 100.0 | 81.6 | 55.7 | 58.9 | 53.1 |
| 0.5% HEC + Met 20mM | 100.0 | 104.0 | 59.9 | N/D | N/D |
| 0.5% HEC + Gly 20mM | 100 | 83.2 | N/D | N/D | N/D |
| HSA+NaCl | 100.0 | 112.7 | 86.0 | 61.5 | 45.6 |
| Sodium Phosphate(pH6.0) | 100.0 | 105.2 | 89.4 | 67.9 | - |

According to the experiment result, it was confirmed that, compared with the positive control HSA, the condition of using PVP or HEC as a thickening agent and a disaccharide or methionine as a stabilizer was the optimally stabilized combination for preparing a botulinum toxin coating composition.

### Example 6. Confirmation of coating uniformity of coating composition including botulinum toxin

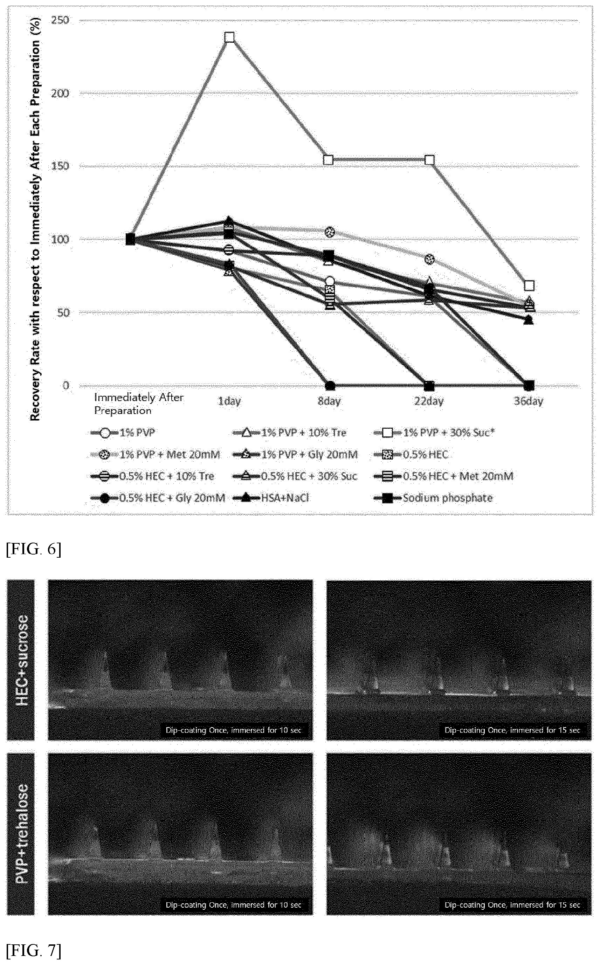

For a composition including botulinum toxin to be coated on a substrate such as a microneedle, as well as the potency stabilization of the botulinum toxin in the composition, how the substrate is uniformly coated with the composition is also very important. Therefore, the composition including a thickening agent+stabilizer combination, evaluated as having excellent botulinum toxin potency stabilization in Example 5, was used to coat a substrate subjected to pretreatment coating with 2% PVA, and coating uniformity was evaluated with an average pixel number and a magenta ratio. More specifically, images were set to CMYK instead of RGB, a needle size was adjusted identically in each image, the background of the images were deleted, except needles, the entire area of the needle part was measured, and the pixel number and magenta ratio of the stained area of the needle part were measured. The results are shown in Tables 8 and 9 and FIG. 6.

**[Table 8]**

| Preparation Example | Average pixel number |
|---|---|
| HEC+Suc, Dip-coating Once, immersed for 10 sec | 392 |
| PVP+Tre, Dip-coating Once, immersed for 10 sec | 366 |
| HEC+Suc, Dip-coating Once, immersed for 15 sec | 605 |
| PVP+Tre, Dip-coating Once, immersed for 15 sec | 402 |

**[Table 9]**

| Preparation Example | Magenta ratio (%, measured 6 times) | | | | | |
|---|---|---|---|---|---|---|
| | Once | Twice | 3 times | 4 times | 5 times | 6 times |
| HEC+Suc, Dip-coating Once, immersed for 10 sec | 84 | 90 | 88 | 92 | 89 | 93 |
| PVP+Tre, Dip-coating Once, immersed for 10 sec | 47 | 81 | 66 | 8 1 | 71 | 79 |

According to the experiment result, when the microneedles were immersed in each composition for 10 seconds or 15 seconds, in all cases, the HEC+Suc combination showed a higher average pixel number compared to the PVP+Tre combination. In addition, when coating was performed for the same period of time, the magenta area ratio (%) with respect to the entire area of microneedles was also significantly high in the HEC+Suc combination (HEC+Suc combination: average 89%, PVP+Tre combination: average 71%). From the above result, it was seen that, when the HEC+Suc combination was used as a coating composition, compared to the PVP+Tre combination, uniform coating was achieved.

### Example 7. Confirmation of optimal mixing ratio of HEC+Suc combination

From Examples 5 and 6, it can be seen that, when coated on the microstructure, the HEC+Suc combination showed excellent viscosity and excellent stabilization compared to the combinations of other thickening agents and stabilizers. Therefore, a test for determining the optimal mixing ratio for the HEC+Suc combination was carried out.

### Example 7-1. Confirmation of viscosity and surface tension by mixing ratio of HEC+Suc combination

The mixing ratios of the HEC+Suc combinations were set in w:w as shown in Table 10, and the remainder other than HEC and sucrose was filled with distilled water and stirred at 200 to 400 rpm for 1 hour or more. The resulting products were sent to the Korean Polymer Testing Laboratory for analysis, and their viscosity and surface tension were measured.

**[Table 10]**

| Preparation Example (name) | (HEC : Sue ratio) | | Concentration (g/mℓ) | |
|---|---|---|---|---|
| | HEC | Sue | HEC | Suc |
| HS0160 | 1 | 60 | 0.005 | 0.300 |
| HS0130 | 1 | 30 | 0.005 | 0.150 |
| HS0110 | 1 | 10 | 0.005 | 0.050 |
| HS0101 | 1 | 1 | 0.005 | 0.005 |
| HS1001 | 10 | 1 | 0.050 | 0.005 |
| HS3001 | 30 | 1 | 0.150 | 0.005 |
| HS6001 | 60 | 1 | 0.300 | 0.005 |

In the process of preparing a composition in various mixing ratios of the HEC+Suc combinations, uniform solutions were able to be prepared with HS0160, HS0130, HS0110, HS0101, and HS1001 using a stirrer. However, from the HS3001 ratio, as a phenomenon of dramatically increasing viscosity to the extent that a magnetic bar in the stirrer may not be stirred occurred, it was impossible to smoothly prepare a composition. Therefore, from the viscosity and surface tension measurement samples, HS3001 and HS6001 formulations were excluded. In preparation, since the HS1001 solution had an excessively high viscosity, compared to other formulations, and surface tension could not be measured, the corresponding formulation was excluded from the surface tension measurement test.

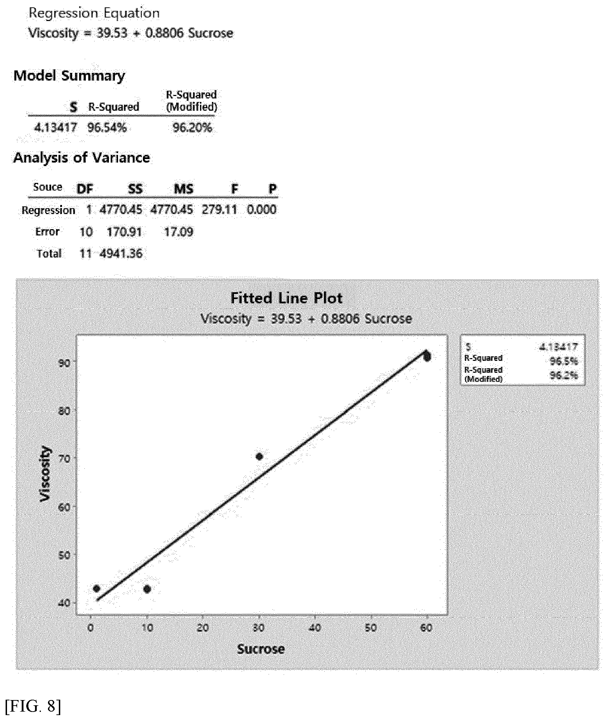

The regression analysis results for the measurement of viscosity and surface tension are shown in FIGS. 7 and 8. As a result of analyzing the viscosity measurement result through one-way ANOVA, it can be seen that the higher the sucrose ratio, the higher the viscosity (R2=96.54%, P=0.000, Viscosity=39.53+0.8806 Sucrose). As a result of measuring the surface tension, it can be also seen through one-way ANOVA that the higher the sucrose ratio, the lower the surface tension (R2=87.27%, P=0.000, Surface tension=64.838-0.05101 Sucrose).

### Example 7-2. Confirmation of coating uniformity by mixing ratio of HEC+Suc combination

Considering the viscosity and surface tension measurement results of Example 7-1, the mixing ratios of HEC+Suc combinations for confirming coating uniformity were set as shown in Table 11 below.

**[Table 11]**

| Preparation Example (name) | HEC : Suc ratio | | Concentration (g/mℓ) | |
|---|---|---|---|---|
| | HEC | Sue | HEC | Suc |
| HS0190 | 1 | 90 | 0.010 | 0.900 |
| HS0180 | 1 | 80 | 0.010 | 0.800 |
| HS0170 | 1 | 70 | 0.010 | 0.700 |
| HS0160 | 1 | 60 | 0.010 | 0.600 |
| HS0150 | 1 | 50 | 0.010 | 0.500 |
| HS0140 | 1 | 40 | 0.010 | 0.400 |
| HS0130 | 1 | 30 | 0.010 | 0.300 |
| HS0110 | 1 | 10 | 0.010 | 0.100 |
| HS0101 | 1 | 1 | 0.010 | 0.010 |

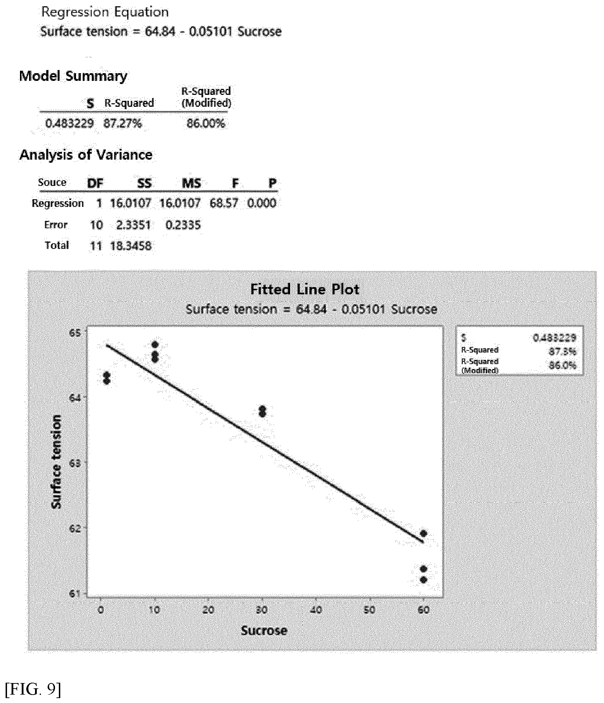

A coating uniformity test was carried out in the same manner as in Example 6. However, in sample preparation, it was easy to prepare HS0101, HS0110, HS0130, HS0160, HS0170, and HS0180 formulations, but in the case of HS0190 formulation, preparation quality was not controlled, and it was concluded that a small amount sucrose was precipitated from a saturated solution the day after preparation, making the formulation unusable. Coating at other ratios was carried out, and images are shown in FIG. 9. As a result of image analysis, it was able to be confirmed that dye coating patterns are irregular at the HS0101 to HS0130 ratios. As the sucrose ratio increased, the dye was more evenly coated, and the color of the dye was stronger.

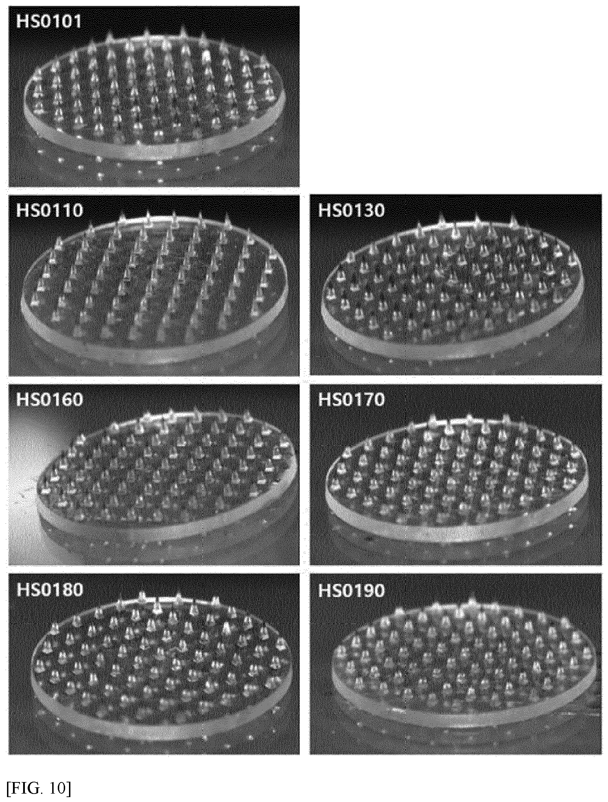

The results of regression analysis for the relative standard deviations of a pixel number from the photographed images are shown in FIG. 10. According to the experiment result, it was confirmed that, in HS0101 with the lowest sucrose ratio, the pixel number was large (5529) in the coated part, but in HS0110 with the next lowest sucrose ratio, the pixel number was the smallest (3682). Afterward, the number of pixels gradually increased according to the sucrose mixing ratio, and in HS0170, the largest number of pixels (5967) was confirmed. Then, as the sucrose content increased, the number of pixels tended to decrease again (HS0180=5207, HS0190=4955). In addition, from the regression analysis result, it can be confirmed that the sucrose mixing ratio enabled the coating pattern between needles in a patch to be uniform.

### Example 7-3. Confirmation of long-term stability by mixing ratio of HEC+Suc combination

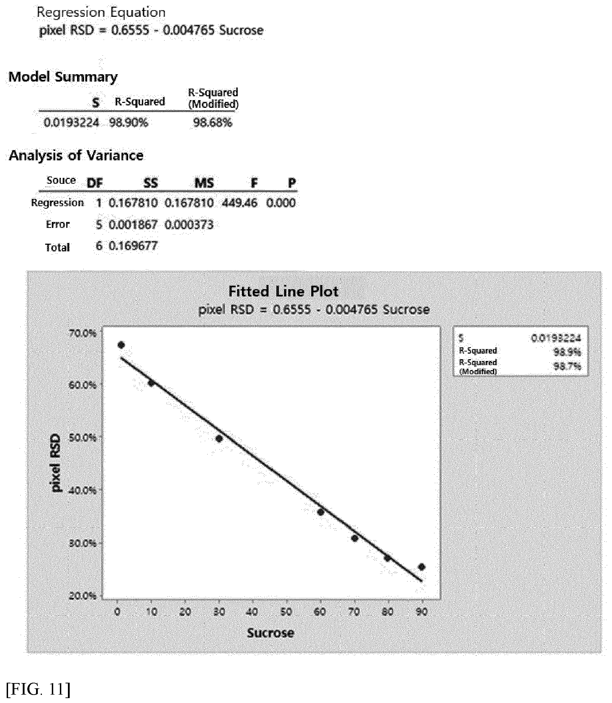

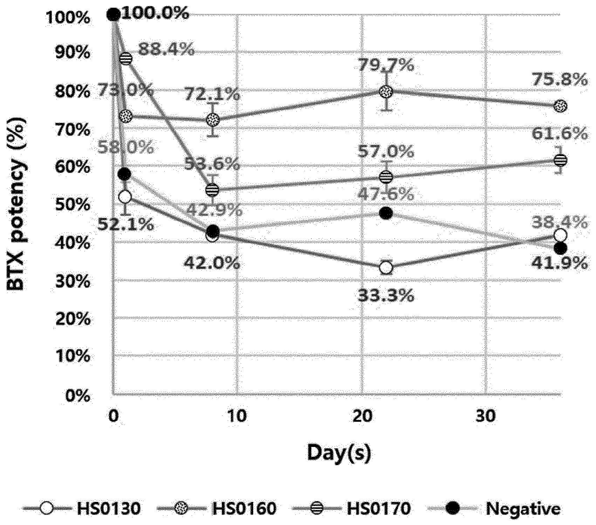

To confirm whether a composition per mixing ratio of HEC+Suc combination can preserve the potency of botulinum toxin on a PLLA patch, the botulinum toxin was measured and applied to the PLLA patch, followed by evaluating accelerated stability at 37 °C for 5 weeks. To this end, a 2X formulation with a HEC:Suc combination in Table 12 was prepared, mixed with botulinum toxin in 1:1, evenly sprayed on a PLLA chip subjected to pretreatment coating with 2% PVA solution, and then completely dried in an incubator at 37 °C. On day 0, 1, 8, 22, and 36, the PLLA chips were collected to measure the potency of the botulinum toxin, and the potency immediately after the formulation of each sample was converted based on 100%, and potency recovery rates for 5 weeks were compared. The result is shown in Table 13 and FIG. 11.

**[Table 12]**

| Preparation Example (name) | HEC : Suc ratio | | Concentration (g/mℓ) | | Note |
|---|---|---|---|---|---|
| | HEC | Suc | HEC | Sue | |
| HS0170 | 1 | 70 | 0.010 | 0.700 | |
| HS0160 | 1 | 60 | 0.010 | 0.600 | |
| HS0130 | 1 | 30 | 0.010 | 0.300 | Positive control |
| Negative | 0 | 0 | 0 | 0 | Negative control |

**[Table 13]**

| Preparation Example (name) | Immediately after preparation | 1day | 8day | 22day 36day | |
|---|---|---|---|---|---|
| HS0170 | 100.0 | 88.4 | 53.6 | 57.0 61.6 | |
| HS0160 | 100.0 | 73.0 | 72.1 | 79.7 | 75.8 |
| HS0130 | 100.0 | 52.1 | 42.0 | 33.3 | 41.9 |
| Negative | 100.0 | 58.0 | 42.9 | 47.6 | 38.4 |

According to the experiment result, it was seen that, in the HS0150 ratio or less, the potency pattern of the botulinum toxin on day 36 is similar to that of the negative control. When a HEC: Suc ratio was 1:30, the potency pattern of the botulinum toxin seemed to be similar to that of the negative control, in the 1:60 and 1:70 experimental groups, compared to the negative control, a botulinum toxin potency of 60% or more (based on day 36) was maintained. From the above result, it can be confirmed that the mixing ratio of hydroxyethyl cellulose and sucrose plays an important role in stability of botulinum toxin, and it can be seen that the HEC+Suc combination ratio for the stability of botulinum toxin should exceed 1:30.

As above, as specific parts of the specification have been described in detail, although it is clear to those skilled in the art that this specific technique is merely a preferred embodiment, the scope of the specification is not limited thereto. Thus, the substantial scope of the specification will be defined by the accompanying claims and their equivalents.

### [Industrial Applicability]

The present invention relates to a microstructure formulation technique for botulinum toxin, which uses a microstructure of the present invention to reduce pain during the administration of botulinum toxin and facilitate the administration of a small amount of toxin at an exact location. Therefore, the present invention is expected to be broadly applied for safe and convenient medical use.

## Claims

1. A composition for coating a microstructure, comprising botulinum toxin, hydroxyethyl cellulose, and sucrose.

2. The composition of claim 1, wherein the botulinum toxin is one or more selected from the group consisting of botulinum toxin types A, B, C, D, E, F, and G.

3. The composition of claim 1, wherein the microstructure is one or more selected from the group consisting of a microneedle, a microblade, a microknife, a microfiber, a microspike, a microprobe, a microbarb, a microarray, and a microelectrode.

4. A microstructure coated with the composition of claim 1.

5. The microstructure of claim 4, wherein the microstructure is one or more selected from the group consisting of a microneedle, a microblade, a microknife, a microfiber, a microspike, a microprobe, a microbarb, a microarray, and a microelectrode.

6. A method of preparing a composition for coating a microstructure, comprising:
mixing botulinum toxin, hydroxyethyl cellulose, and sucrose.

7. The method of claim 6, wherein the botulinum toxin is one or more selected from the group consisting of botulinum toxin types A, B, C, D, E, F, and G.

8. The method of claim 6, wherein the microstructure is one or more selected from the group consisting of a microneedle, a microblade, a microknife, a microfiber, a microspike, a microprobe, a microbarb, a microarray, and a microelectrode.

9. A method of coating a microstructure with a composition comprising botulinum toxin, comprising:
(a) mixing botulinum toxin, hydroxyethyl cellulose, and sucrose; and
(b) coating a microstructure surface with the mixture of (a).

10. The method of claim 9, wherein the botulinum toxin is one or more selected from the group consisting of botulinum toxin types A, B, C, D, E, F, and G.

11. The method of claim 9, wherein the microstructure is one or more selected from the group consisting of a microneedle, a microblade, a microknife, a microfiber, a microspike, a microprobe, a microbarb, a microarray, and a microelectrode.
